# EUROPEAN PATENT SPECIFICATION

(11) **EP 3 687 991 B1**
(45) Date of publication and mention of the grant of the patent: **05.07.2023**
(21) Application number: 18785280.1
(22) Date of filing: 28.09.2018
(51) Int. Cl.: B01D 57/02, C07D 417/06, C09B 23/02, C12Q 1/6816, G01N 27/447, G01N 33/58

(54) **MULTICOMPONENT TABLET FOR MAKING AGAROSE GEL AND METHODS THEREOF**
MEHRKOMPONENTEN TABLETTE ZUR HERSTELLUNG EINES AGAROSE GELS UND VERFAHREN DAFÜR
COMPRIME MULTICOMPOSANT POUR LA PREPARATION D'UN GEL D'AGAROSE ET PROCEDE ASSOCIE

(30) Priority: 30.09.2017 US 201762566350 P
(43) Date of publication of application: 05.08.2020
(73) Proprietor: Bioatlas OÜ, 51014 Tartu (EE)
(72) Inventor: VIRU, Liane, 50111 Tartu Tartumaa (EE); KASK, Indrek, 76905 Muraste Harjumaa (EE); KOORITS, Lauri, 50412 Tartu Tartumaa (EE); EINASTE, Taavi, 11412 Tallinn Harjumaa (EE)
(74) Representative: Aera A/S
(86) International application number: PCT/EP2018/076478
(87) International publication number: WO 2019/063795

(56) References cited:
- EP-A1- 1 335 027
- WO-A1-2005/033342
- US-B1- 8 877 437

## Description

### FIELD

The present invention relates to compressed compositions for use in electrophoretic analysis comprising polymer matrix particles and lyophilized fluorescent DNA-binding dye, and/or compressed compositions for use in electrophoretic analysis comprising a blend of polymer matrix particle(s) and electrophoretic conductive medium particle(s) having a uniform particle size and lyophilized fluorescent DNA-binding dye.

### BACKGROUND

Gel electrophoresis is a method for separation and analysis of macromolecules (DNA, RNA and proteins) and their fragments, based on their size and charge. It is used, for example, in clinical biochemistry to separate proteins and in molecular biology to separate a mixed population of DNA and RNA fragments by length, to estimate the size of DNA and RNA fragments or to separate proteins by charge, and electrophoresis has been widely used since the 1960s.

Traditionally, the preparation of a gel for electrophoresis included manually preparation of stock solutions including for example weighing agarose and buffer powders individually and again manually heat the solution and add various dyes to the heated solution prior to use. Later agarose and buffer blends emerged, like for example Sigma-Aldrich's "Agarose - TBE Blend 1.0%", where one only would have to make one weighing of the blend and mix with water. According to the manufactures' description, one should e.g. weigh out 2.703 g of Agarose-TBE blend 1.0 % into a beaker or a flask and add 100 mL of deionized water. However, weighing mistake often happens in such a manual process, and therefore the separation quality diminishes, and of course such manual process is obviously time consuming.

Furthermore, the powders or mixtures are typically granulated, and air movement around, into or out of granular or powdered material will disperse dust. Therefore, handling methods for bulk materials, such as filling and emptying flasks or transferring materials from one holder to another, may constitute appreciable dust sources, which are dangerous to the respiratory tract.

Furthermore, the manual addition and evaporation of the dye, like Ethidium bromide used to detect nucleic acids, provides a significant health risk as most dyes used in electrophoresis are carcinogenic.

Thus, there is a need for improved compositions for use in electrophoretic analysis.

### SUMMARY

In one aspect, the invention relates to a compressed composition for use in electrophoretic analysis comprising polymer matrix particles, and lyophilized fluorescent DNA-binding dye. The compressed composition, or tablet, is easy to use and on top provides a significant improvement over the traditional health risks found in traditional electrophoretic analysis preparations.

The composition of the present invention is defined in claim 1.

According to the present invention the polymer matrix particles comprise agarose.

The compressed composition of the invention is ideal for routine DNA and RNA gel electrophoresis and blotting assays because:
Tablet format - no weighing equipment required
Powder free
Very stable at room temperature if protected from light
Only pure water is needed
Fast dissolving protocol
Safer DNA stain
Environmental safety - all working solutions can be disposed in regular laboratory waste* (*according to local regulation)

In another aspect, the invention relates to a compressed composition for use in electrophoretic analysis comprising a blend of polymer matrix particle(s) and electrophoretic conductive medium particle(s) having a uniform particle size, and lyophilized fluorescent DNA-binding dye.

A presently preferred embodiment of invention relates to a compressed composition, wherein the composition consists of the polymer matrix particles, the lyophilized fluorescent DNA-binding dye and/or the electrophoretic conductive medium particles.

In a third aspect, the invention relates to a method for preparing a compressed composition for use in electrophoretic analysis comprising:
a) providing a blend of polymer matrix particles comprising agarose and buffers made from electrophoretic conductive medium particles, wherein the blend has a uniform particle size, a lyophilized fluorescent DNA-binding dye and a cryoprotectant, and
b) compressing the blend obtained in step a).

The method of the present invention is defined in claim 4.

A method for preparing a lyophilized dye for use in a compressed composition, typically comprises
a) providing a fluorescent DNA-binding dye in liquid format and a cryoprotectant, and
b) lyophilization the fluorescent DNA-binding dye and cryoprotectant by use of low temperature, pressure and sublimation.

### DETAILED DESCRIPTION

The materials necessary to perform electrophoresis cost little and are fairly easy to prepare. Supply cost is one factor in the expense of laboratory testing, but it is by far the time spent by the technicians that endures the most costs. Furthermore, of all the benefits electrophoresis offers, reliability is the most essential. If done correctly, electrophoresis will separate all molecules in a specimen. For a lab technician, the ability to rely on a technique makes their job easier and allows for effective results. DNA testing and analysis is a complicated process, but one that allows for forensic identifications, species analysis and diagnosis of diseases. Electrophoresis helps make that happen.

Thus, by minimizing the time consumption in the labs, especially on routine work, efficiency gains are huge and at the same time maintaining or even improving reliability is what most laboratories strive for.

The compressed composition for use in electrophoretic analysis, as described herein, achieves amongst other benefits this by providing an easy-to-use tablet containing everything necessary for an easy preparation of an agarose gel in desired percentages. For example, to make a various % agarose gels, the technician would just have to add one or two tablets of e.g. the currently preferred tablet with TAE according to the following table:

| Gel % | 1 tablet | 2 tablets |
|---|---|---|
| 0.8 % | 81 ml water | 162 ml water |
| 1 % | 65 ml water | 130 ml water |
| 1.2 % | 54 ml water | 108 ml water |
| 1.5 % | 43 ml water | 86 ml water |
| 2% | 32.51 ml water | 65 ml water |

The compressed composition is optimized to yield high resolution of e.g. sharp DNA bands with high sensitivity and low background. The compressed composition is compatible with a wide variety of gel reading instruments.

### Compressed composition

In one or more exemplary embodiments, the invention is a compressed composition for use in electrophoretic analysis comprising polymer matrix particles, and lyophilized fluorescent DNA-binding dye.

In one or more exemplary embodiments, the invention is a compressed composition for use in electrophoretic analysis comprising a blend of polymer matrix particle(s) and electrophoretic conductive medium particle(s) having a uniform particle size, and lyophilized fluorescent DNA-binding dye.

In the present context, the compressed composition may for example be a tablet. Tablets in the present context may be defined as the solid unit dosage form of the components with or without suitable excipients and prepared either by moulding or by compression. It can comprise a mixture of the components and excipients, usually in powder form, pressed or compacted from a powder into a solid dose.

In one or more exemplary embodiments, the compressed composition for use in electrophoretic analysis is prepared by direct compression.

### Ratio between the agarose and the buffer - blends

The invention provides a ratio between the agarose and the buffer inside the compressed composition, this ratio has several benefits:
- maintains the required agarose gel pH level
- maintains good buffering capacity, and
- maintains good resolution and sharp DNA bands

For example, a very good resolution can be achieved by using 0.5 × TBE to 1.5 × TBE at voltages up to 250 V (25 V per cm). It is even similar when using a TAE buffer.

In one or more exemplary embodiments, the compressed composition is a blend of:
- 40 to 75 wt % agarose powder;
- 25 to 60 wt % DNA electrophoretic conductive medium;
- less than 0.05 wt%, lyophilized fluorescent DNA-binding dye.

Thus, the compressed composition may in one or more exemplary embodiments be a blend of:
- 0.65 g Agarose + 0.35 g TAE + lyophilized fluorescent DNA-binding dye (2.6µl DNA binding Dye + 0.26 mg cryoprotectant), which equals to:
   - 65 wt % agarose powder;
   - 35 wt % DNA electrophoretic conductive medium;
   - less than 1 wt % lyophilized fluorescent DNA-binding dye
      or
      0.5 g Agarose + 0.5 g TAE + lyophilized (2µl lyophilized fluorescent DNA-binding dye + 0.2 mg cryoprotectant), or
      0.5 g Agarose + 0.5 g TBE+ lyophilized (2µl lyophilized fluorescent DNA-binding dye + 0.2 mg cryoprotectant), which equals to:
         - 50 wt % agarose powder;
         - 50 wt % DNA electrophoretic conductive medium;
         - less than 1 wt % lyophilized fluorescent DNA-binding dye
            or
      0.5 g Agarose + 0.5 g TBE + lyophilized (2µl DNA binding dye + 0.2 mg cryoprotectant), which equals to:
         - 50 wt % agarose powder;
         - 50 wt % DNA electrophoretic conductive medium;
         - less than 1 wt % lyophilized fluorescent DNA-binding dye
            or
      0.65 g Agarose + 0.35 g TBE + lyophilized (2.668 µl DNA binding dye + 0.2668 mg cryoprotectant), which equals to:
         - 65 wt % agarose powder;
         - 35 wt % DNA electrophoretic conductive medium;
         - less than 1 wt % lyophilized fluorescent DNA-binding dye

The compressed composition may in one or more presently preferred exemplary embodiments be a blend of:
- a lyophilized DNA binding dye of 0.004 % of the final volume with a cryo-preservative of 5-10 % DNA-binding dye and solvent residues in the range of 0.1-0.3 % of the final binding volume of DNA
- crushed acrylic buffer 35 to 50 %;
- agarose 50 to 65 %.

In the present context, a 2 µl lyophilized DNA binding dye means: 0.26 mg cryoprotectant + 0.00512 mg mixed lyophilized DNA binding dye and some HCL residue and water molecules.

In one or more presently preferred exemplary embodiments, the compressed composition for use in electrophoretic analysis comprises a blend of polymer matrix particle(s) and electrophoretic conductive medium particle(s) having a uniform particle size, and lyophilized fluorescent DNA-binding dye, wherein the blend has a uniform particle size between 20-300 µm and wherein the blend comprises between:
- 40 to 75 wt % agarose powder;
- 25 to 60 wt % DNA electrophoretic conductive medium;
- less than 1 wt % lyophilized fluorescent DNA-binding dye

In one or more exemplary methods, the dye may be lyophilized prior to direct compression.

The lyophilization parameters are, for example, 0.07 mBar with a final dry at 0.011 mBar.

In one or more exemplary embodiments, the compressed composition reduces the amount of dust or powdered form of the polymer matrix particles in the laboratory, which makes the use safer especially for the lab technician working with a high volume of routine electrophoretic analysis.

The compressed compositions according to the invention is subjected to high quality control, and all tablets are precision weighed prior to shipment. In one or more exemplary embodiments, the compressed composition reduces potential weighing errors.

Weighing errors often occurs because two separate weights must be performed (agarose and conductive media). This will endure 2 scale errors (usually 0.01 %), and when these weighing errors take place, parts of the weighed material remain in the weighing container, which further adds up. This combined with typical human error like simply wrong weighing or rounding errors makes the typical use difficult.

Furthermore, when making conductive media, fluid errors occur. Like errors from beaker/measuring glass, combined with human errors.

When adding the DNA-binding dye, typical pipetting errors often occurs.

In one or more exemplary embodiments, the compressed composition is easily dissolved in water at or below room temperature. The compressed composition is simply dissolved with the desired water amount for 1-3 minutes before heating.

In one or more exemplary embodiments, the compressed composition is easily dissolved in water at or below room temperature in less than 5 minutes, such as less than 4 minutes, less than 3 minutes, less than 2 minutes or less than 1 minute.

In one or more exemplary embodiments, the compressed composition reduces the amount of polymer matrix particles in dust or powdered form in the laboratory, which makes the use safer.

In one or more exemplary embodiments, the compressed composition is very stable in room temperature. The tablets are protected from light by packaging and this is advantageous because typical liquid DNA-binding dyes are usually not stable at room temperature.

In one or more exemplary embodiments, the compressed composition is stable even if the compressed composition is not protected from light by packaging, because agarose and conductive media particles are protecting or shielding the lyophilized fluorescent DNA-binding dye particles from light.

In one or more exemplary embodiments, the compressed composition saves lab worker time.

### Excipients

Compressed composition often comprises excipients. The excipients can include diluents, binders or granulating agents, glidants (flow aids) and lubricants to ensure efficient tabletting; disintegrants to promote tablet break-up and pigments to make the tablets visually attractive or aid in visual identification of an unknown tablet. A polymer coating can be applied to control the release rate of components, to make it more resistant to the environment (extending its shelf-life), or to enhance the tablet's appearance.

In one or more exemplary embodiments, the compressed composition for use in electrophoretic analysis is prepared comprising suitable excipients.

Suitable excipients may for example be binders, lubricants, glidants, anti-adherents and/or super disintegrants.

Examples of binders are gelatine, glucose, lactose, cellulose derivatives such as methyl cellulose, ethyl cellulose, hydroxy propylmethyl cellulose, hydroxy propyl cellulose, starch, poly vinyl pyrrolidone (Povidone), sodium alginate, carboxymethylcellulose, acacia etc.

Examples of lubricants are insoluble lubricants such as stearic acid, magnesium stearate, calcium stearate, talc or talcum, and paraffin or soluble lubricants such as sodium lauryl sulphate, sodium benzoate, PEG 400, 600, 8000 etc.

Examples of glidants are Colloidal Silicon dioxide (Aerosil), Corn-starch, Talc etc.

Examples of anti-adherents includes talc or talcum.

Examples of super disintegrants are Croscarmellose sodium (Ac-di-sol), Crospovidone (Polyplasdone), sodium starch glycollate, starch etc.

However, the additive substances or excipients can affect the movement of macromolecules like DNA or RNA in the agarose gel. Thus, in one or more exemplary embodiments, the compressed composition for use in electrophoretic analysis is prepared without suitable excipients. Exemplary embodiments of the invention also include barely traceable amounts of excipients.

Exemplary embodiments of the invention also include compressed composition for use in electrophoretic analysis, wherein the amounts of excipients are less than 0.5 % w/w, such as but no limited to less than 0.4 % w/w, less than 0.3 % w/w, less than 0.2 % w/w, less than 0.1 % w/w, less than 0.05 % w/w, less than 0.005 % w/w, less than 0.0005 % w/w, less than 0.0001 % w/w, and less than 0.00001 % w/w.

### The polymer matrix particles

A polymer matrix particle according to the invention can be any matrix forming polymer, which is able to separate a charged target macro molecule, such as but not limited to DNA, RNA, oligonucleotides and proteins. So, in the present context, a polymer matrix particle can be a three-dimensional matrix formed of a polymer that aggregate into a three-dimensional structure with channels and pores through which macro molecule can pass. This enables macro molecule to be separated by size.

Examples of polymer matrix particle(s) according to the invention can for example be selected from the group consisting of agarose, polyacrylamide and starch.

In one or more exemplary embodiments, the polymer matrix particle is agarose.

In one or more exemplary embodiments, the polymer matrix particle is polyacrylamide.

In one or more exemplary embodiments, the polymer matrix particle is starch.

### The electrophoretic conductive medium particles

Electrophoretic conductive medium in gel electrophoresis are used to provide ions that carry a current and to maintain the pH at a relative constant value. The electrophoretic conductive medium or buffers is/are made by electrophoretic conductive medium particle(s) dissolved typically in water. These buffers have plenty of ions in them, which is necessary for the passage of electricity through them, unlike distilled water.

There are a number of buffers used for electrophoresis. The most common being, for nucleic acids Tris/Acetate/EDTA (TAE), Tris/Borate/EDTA (TBE). Many other buffers have been proposed, e.g. lithium borate, iso electric histidine, pK matched goods buffers, etc.

The electrophoretic conductive medium particle(s) of the present invention is preferably powdered TBE and TAE.

In one or more exemplary embodiments, the electrophoretic conductive medium particle is TRIS based.

In one or more exemplary embodiments, the electrophoretic conductive medium particle is TAE.

In one or more exemplary embodiments, the electrophoretic conductive medium particle is TBE.

### Similar Size

It is advantageous that the blend of the polymer matrix particle and the DNA electrophoretic conductive medium particle have a uniform particle size. As shown in Example 6, the compressed composition according to the invention, remains intact and its shape is preserved, while a non-uniform size shows cracks.

Therefore, the size of the polymer matrix particle component in the compressed composition, according to the invention, should be similar or uniform to that of the electrophoretic conductive medium particle, because it is otherwise difficult to obtain a homogeneous composition. A non-uniform size of the components in the compressed composition also affects the efficacy of tablet formation.

If a homogeneous composition is not obtained it results in non-optimal concentration of electrophoretic conductive medium, which lowers the electrophoretic conductive medium's capacity. Thus, the DNA migration in agarose gel made of non-uniform size particle compressed composition is complicated.

In one or more exemplary embodiments, the compressed composition according to the invention is a homogeneous composition.

Blends having small particles size do not easily form compressed composition without additives, and blends having large particles do not meet the requirements for the application or use of the compressed composition according to the present invention, for example they may be insoluble.

Thus, in one or more exemplary embodiments, the blend has a uniform particle size between 20-300 µm, being the size prior to compression.

In one or more exemplary embodiments, the compressed composition according to the invention is a compressed composition containing 0.5 g uniform particle size polymer matrix particle, 0.5 g uniform particle size electrophoretic conductive medium TBE and 2 µl lyophilized fluorescent DNA-binding dye mixed with 0.2 mg cryoprotectant mannitol.

The 1 % agarose gel solution made of this compressed composition contains 50 ml water, 0.5 g polymer matrix particle, 0.5 g electrophoretic conductive medium TBE (which corresponds to a concentration of 0.59-fold liquid conductive medium), 2 µl fluorescent DNA-binding dye and 0.2 mg mannitol.

In one or more exemplary embodiments, the compressed composition, according to the invention, is a compressed composition with TAE containing 0.5 g uniform particle size polymer matrix particle, 0.5 g uniform particle size electrophoretic conductive medium TAE and 2.6 µ! lyophilized fluorescent DNA-binding dye mixed with 0.26 mg cryoprotectant mannitol.

The 1% agarose gel solution made of this compressed composition contains 65 ml water, 0.65 g polymer matrix particle, 0.35 g electrophoretic conductive medium TAE (which corresponds to a concentration of 0.84-fold liquid conductive medium), 2.6 µl fluorescent DNA-binding dye and 0.2 mg mannitol.

### Target molecule

Gel electrophoresis is a method for separation and analysis of macromolecules (DNA, RNA and proteins) and their fragments, based on their size and charge. Thus, the compressed compositions when used in gel electrophoresis is capable of identifying a target molecule. The target molecule is for example RNA, DNA, protein, and/or oligonucleotides.

In one or more exemplary embodiments, the compressed compositions are free of contaminants such as but not limited to nonspecific DNase, nonspecific RNase, or protease.

### Fluorescent DNA-binding dye

There are several different fluorescent DNA-binding dyes or stains that can be used to visualize macromolecules such as e.g. DNA after the material has been separated by gel electrophoresis. Among the more than 50 choices available on the market, the most common is Ethidium Bromide. However, when working with gel electrophoresis, it is important to not only know the technical differences between the various dyes but also the inherent health risks. Over the years, health concerns have arisen in particular about Ethidium Bromide's role as a mutagen, due to it being an intercalating agent. This has spurred intense debate as to whether fluorescent DNA-binding dyes like Ethidium Bromide is optimal for conventional laboratory use.

Fluorescence in the present context is the emission of light by a substance that has absorbed light or other electromagnetic radiation, and fluorescent DNA-binding dye according to the invention uses a non-destructive way of tracking or analyzing biological molecules.

The dye in the compressed compositions according to the invention may also detect other macromolecules like RNA and/or protein.

In one or more exemplary embodiments, the fluorescent DNA-binding dye is a colored substance that has an affinity to the macromolecule to which it is being applied. The dye may for example be selected from the group consisting of Acridine Orange, DAPI, cyanine dye (TOTO, YOYO, Stains All and others), Ethidium Bromide (EtBr), Coomassie Brilliant Blue, GelGreen, GelRed, SYBR Green I, propidium iodide, Hoechst, PicoGreen, Thiazole orange or mixtures thereof.

Some dyes like Thiazole Orange has been shown to be three to four times less mutagenic than Ethidium Bromide, and others like for example SYBR Safe is four to five less mutagenic. So, to reduce the carcinogenic effects, then in one or more exemplary embodiments the dye is selected from the group consisting of Acridine Orange, DAPI, GelGreen, GelRed, SYBR Green I, SYBR Safe, Thiazole Orange or mixtures thereof.

In one or more exemplary embodiments, the dye is selected from the group consisting of Acridine Orange, DAPI, GelGreen, GelRed, SYBR Green I, SYBR Safe, Thiazole Orange or mixtures thereof.

In one or more exemplary embodiments, the dye is selected from the group consisting of SYBR Green I, propidium iodide, Hoechst, PicoGreen, Thiazole Orange or mixtures thereof.

In one or more exemplary embodiments, the dye is selected from the group consisting of Acridine Orange, DAPI, SYBR Safe, Thiazole Orange or mixtures thereof.

In one or more exemplary embodiments, the dye is selected from the group consisting of GelGreen, GelRed, SYBR Green I, SYBR Safe or mixtures thereof.

In one or more exemplary embodiments, the dye is selected from the group consisting of GelGreen, SYBR Green I or mixtures thereof.

In one or more exemplary embodiments, the dye is selected from the group consisting of Ethidium Bromide (EtBr), GelRed or mixtures thereof.

In one or more exemplary embodiments, the dye is selected from the group consisting of Acridine Orange, DAPI, SYBR Safe or mixtures thereof.

In one or more exemplary embodiments, the dye is selected from the group consisting of Acridine Orange, DAPI or mixtures thereof.

In one or more exemplary embodiments, the dye is selected from the group consisting of Acridine Orange, Thiazole Orange or mixtures thereof.

In one or more exemplary embodiments, the dye is selected from the group consisting of DAPI, Thiazole Orange or mixtures thereof.

These combinations bind better to e.g. DNA and therefore show a brighter band and have less background.

### Acridine Orange

Acridine Orange (3-*N*,3-*N*,6-*N*,6-*N*-Tetramethylacridine-3,6-diamine) is a dye that intercalates or binds with the nucleic acids. This binding is the result the electrostatic interactions of acridine molecule between the nucleic acid base pairs. When bound to DNA, it is very similar spectrally to fluorescein, with an excitation maximum at 502 nm and an emission maximum at 525 nm (green). When it associates with RNA, the excitation maximum shifts to 460 nm (blue) and the emission maximum shifts to 650 nm (red).

In one or more exemplary embodiments, the dye is Acridine Orange.

### DAPI

DAPI (4',6-diamidino-2-phenylindole) is a fluorescent stain that binds strongly to A-T rich regions in DNA. When bound to double-stranded DNA DAPI has an absorption maximum at a wavelength of 358 nm (ultraviolet) and its emission maximum is at 461 nm (blue). DAPI will also bind to RNA, though it is not as strongly fluorescent. Its emission shifts to around 500 nm when bound to RNA.

In one or more exemplary embodiments, the dye is DAPI.

### GelGreen

GelGreen also known as GelGreen 10, 10'-(6,22-dioxo-11,14,17-trioxa-7,21-diazaheptacosane-1,27-diyl)bis(3,6-bis(dimethylamino)acridin-10-ium) iodide. GelGreen is an also intercalating nucleic acid stain and is structurally closely related to acridinium orange and consists of two acridinium orange subunits that are bridged by a linear spacer.

Its fluorophore, and therefore its optical properties, are essentially identical to those of other N-alkylacridinium orange dyes. When exposed to ultraviolet light, it will fluoresce with a greenish color that strongly intensifies after binding to DNA. GelGreen is compatible with either a 254 nm UV transilluminator or a gel reader equipped with visible light excitation such as blue light 488 nm. GelGreen can be as a solution in DMSO or in water.

In one or more exemplary embodiments, the dye is GelGreen.

### GelRed

GelRed also known as 5,5'-(6,22-dioxo-11,14,17-trioxa-7,21-diazaheptacosane-1,27-diyl)bis(3,8-diamino-6-phenylphenanthridin-5-ium) iodide, is an intercalating nucleic acid stain. GelRed is structurally closely related to Ethidium Bromide and consists of two ethidium subunits that are bridged by a linear spacer.

Its fluorophore, and therefore its optical properties, are essentially identical to those of Ethidium Bromide. When exposed to ultraviolet light, it will fluoresce with an orange colour that strongly intensifies after binding to DNA. GelRed can be as a solution in DMSO or in water.

In one or more exemplary embodiments, the dye is GelRed.

### Cyanine dye (YOYO, TOTO)

Cyanine is the non-systematic name of a synthetic dye family belonging to a polymethine group. Cyanine dye can advantageously replace conventional dyes such as fluorescein and rhodamines because they are yielding brighter and more stable fluorescence. Cy3 and Cy5 are the most popular, used typically combined for two colors detection.

Cy3 fluoresces greenish yellow (-550 nm excitation, ~570 nm emission), while Cy5 is fluorescent in the red region (-650 excitation, 670 nm emission).

Other cyanine originating nucleic acid dyes SYTO-13, TOTO-1, YOYO-1 Cy3.5, Cy5.5, Cy7, and Cy7.5 dye is also useful.

In one or more exemplary embodiments the dye is Cyanine dye.

### SYBR Green I

SYBR Green I also know as N',N'-dimethyl-N-[4-[(E)-(3-methyl-1,3-benzothiazol-2-ylidene)methyl]-1-phenylquinolin-1-ium-2-yl]-N-propylpropane-1,3-diamine.

SYBR Green I is an asymmetrical cyanine dye used as a nucleic acid stain in molecular biology. The SYBR Green I and other SYBR family of dyes bind to DNA. The resulting DNA-dye-complex absorbs blue light (Amax = 497 nm) and emits green light (λmax = 520 nm). The stain preferentially binds to double-stranded DNA, but will stain single-stranded DNA with lower performance. SYBR Green can also stain RNA with a lower performance than DNA.

In one or more exemplary embodiments, the dye is SYBR Green I.

### SYBR Safe

SYBR Safe, also know as (Z)-4-((3-Methylbenzo[d]thiazol-2(3H)-ylidene)methyl)-1-propylquinolin-1-ium 4-methylbenzenesulfonate, is a cyanine dye used as a nucleic acid stain. SYBR Safe is one of a number of SYBR dyes made by the Life Technologies Corporation. SYBR Safe binds to DNA.

The resulting DNA-dye-complex absorbs blue light (λmax = 509 nm) and emits green light (λmax = 524 nm).

In one or more exemplary embodiments, the dye is SYBR Safe.

### Propidium iodide

Propidium iodide has the chemical formula C27H34I2N4 and is a fluorescent intercalating agent that can be used to stain cells. Propidium iodide binds to DNA by intercalating between the bases with little or no sequence preference. In aqueous solutions, the dye has an excitation/emission maxima of 493/636 nm. Once the dye is bound, its fluorescence is enhanced 20- to 30-fold, the fluorescence excitation maximum is shifted -30-40 nm to the red and the fluorescence emission maximum is shifted -15 nm to the blue, resulting in an excitation maximum at 535 nm and a fluorescence emission maximum at 617 nm.

In one or more exemplary embodiments, the dye is propidium iodide.

### Hoechst

Hoechst is also known as 33342 2'-(4-Ethoxyphenyl)-5-(4-methyl-1-piperazinyl)-2,5'-bi-1H-benzimidazole trihydrochloride. Hoechst stains are part of a family of blue fluorescent dyes used to stain DNA. These Bis-benzimides were originally developed by Hoechst AG, which numbered all their compounds so that the dye Hoechst 33342 is the 33,342nd compound made by the company. There are three related Hoechst stains: Hoechst 33258, Hoechst 33342, and Hoechst 34580. The dyes Hoechst 33258 and Hoechst 33342 are the ones most commonly used, and they have similar excitation-emission spectra.

Both dyes are excited by ultraviolet light at around 350 nm, and both emit blue-cyan fluorescent light around an emission spectrum maximum at 461 nm. Unbound dye has its maximum fluorescence emission in the 510-540 nm range. Hoechst stains can be excited with a xenon- or mercury-arc lamp or with an ultraviolet laser. Hoechst dyes are soluble in water and in organic solvents such as dimethyl formamide or dimethyl sulfoxide.

In one or more exemplary embodiments, the dye is Hoechst

### PicoGreen

Pico488 (Molecular Probes, Inc., Eugene, Oreg) is a DNA binding benzothiazole dye whose structure is identical to PicoGreen. This reagent is a 200× solution in DMSO. The dye is a fluorescent sensor that is specific for double stranded DNA (dsDNA).

In one or more exemplary embodiments, the dye is PicoGreen.

### Thiazole Orange

Thiazole Orange 1-Methyl-4-[(3-methyl-2(3H)-benzothiazolylidene)methyl]quinolinium p-tosylate Thiazole Orange can be tethered with peptide nucleic acid for the detection of target nucleic acid in homogeneous solution. It is a dye with both predicted physical characteristics and the biological staining characteristics of a good reticulocyte dye, hence it is considered as a dye for reticulocyte analysis. It has massive fluorescence enhancement and high quantum yield upon binding to nucleic acids, particularly RNA. Thiazole Orange excitation/emission maxima is 512/533 nm (with DNA).

In one or more exemplary embodiments, the dye is Thiazole Orange.

In one or more presently preferred exemplary embodiments, the dye is Atlas ClearSight dye.

### Lyophilized fluorescent DNA-binding dye

The pharmaceutical industry uses freeze-drying to produce tablets or wafers, the advantage of which is less excipient as well as a rapidly absorbed and easily administered dosage form. By removing the water from the material, the material can easier be stored, shipped, and later reconstituted to its original form. When the dried form is reconstituted in an aqueous system, it may be redispersed to achieve its original particle size. The redispersibility depends on the parameters of the freeze-drying process.

Freeze-drying in the present context, also known as lyophilization or cryodesiccation, is a low temperature dehydration process which involves freezing the product, lowering pressure, and then removing the ice by sublimation. This is in contrast to dehydration by most conventional methods that evaporate water using heat. Freeze-drying results in a high-quality product because of the low temperature used in processing. The original shape of the product is maintained, and quality of the rehydrated product is excellent.

### Method of producing a lyophilized fluorescent DNA-binding dye

In one or more exemplary embodiments, the invention also relates to a method for preparing a lyophilized fluorescent DNA-binding dye for use in a compressed composition as described herein comprising:
a) providing a fluorescent DNA-binding dye in liquid format and a cryoprotectant, and
b) lyophilization of the fluorescent DNA-binding dye and cryoprotectant by use of low temperature, pressure and sublimation.

### The lyophilization parameters.

In one or more exemplary embodiments, the invention also relates to a method for preparing a lyophilized fluorescent DNA-binding dye wherein the lyophilization is performed at 0.07-0.0011 mBar for at least 10 hours.

In one or more exemplary embodiments, the invention also relates to a method for preparing a lyophilized fluorescent DNA-binding dye wherein the final dry is performed at 0.07-0.0011 mBar for at least 1 hour.

In one or more exemplary embodiments, a fluorescent DNA-binding dye in liquid format and a cryoprotectant solution was first frozen at -80 °C) and subsequently dry-frozen at 0.07-0.0011 mbar for 17 hours. The resulting solid was mixed with agarose powder (0.8% of the final volume) and grinded for 2 min prior to use in a blend.

In generic terms the lyophilized fluorescent DNA-binding dye according to the invention may be prepared by mixing the fluorescent DNA-binding dye with a cryo-protectant and frozen to -80 degrees. A presently preferred freezing process is to freeze the fluorescent DNA-binding dye and cryo-protectant as fast as possible by using liquid nitrogen and then placed them in a minus 80° C freezers for 2 hours or until the components are completely frozen.

Then, the frozen components are lyophilized by using parameters settings as main drying at 0.07 mBar for 16 hours and then final drying at 0.011 mBar 2-3 hours.

It is advantageous to have the liquid frozen quickly, because the structure is maintained and the frost damage is the smallest.

The addition of Mannitol reduces the freezing point. This means that the required pressure can be lower. This is also advantageous because it reduces the loss of dye component due to sublimation.

It is important to note that the parameters of the freeze-drying process above may be slightly altered, but to maintain the redispersibility in an optimal condition, not too much. The applicant for example shows the effect of cryoprotectants on the redispersibility as a function of freezing rate.

A fast freezing rate and a high cryoprotectant concentration were favoured. However, under certain conditions, a slower freezing rate resulted in better redispersibility. This is probably because slow freezing can produce a more cryo-concentrated liquid phase, and the concentrated cryoprotectant in the liquid phase can more effectively protect particles.

The redispersibility did not correlate straightforwardly with either the freezing rate or cryoprotectant concentration. Within the whole range of freezing rate and cryoprotectant concentrations, a faster freezing rate and a higher concentration were generally better. However, an adverse effect of cryoprotectant addition was found in many cases. In a redispersibility map of freezing rate and concentration, a region of poor redispersibility was found. At a relatively slow freezing rate region, a slower freezing rate resulted in a lower aggregation of particles.

Slow freezing could lead cryoprotectants to fully migrate into a cryo-concentrated liquid phase, resulting in a high local concentration of cryoprotectant.

The high concentration could effectively prevent the aggregation of particles.

In one or more exemplary embodiments, the cryoprotectant is mannitol and the preferred concentration is 10-18.18 % w/v.

In one or more exemplary embodiments, the lyophilization process uses at least one cryoprotectant in a concentration range of 10 to 18.18 % w/v.

In one or more exemplary embodiments, the lyophilization method comprises a mixture of more than one fluorescent DNA-binding dye and at least one cryoprotectant.

### HCL

Hydrogen chloride is a hydrogen halide. At room temperature, it is a colourless gas, which forms white fumes of hydrochloric acid upon contact with atmospheric water vapor. Hydrochloric acid, the aqueous solution of hydrogen chloride, is also commonly given the formula HCl.

Before lyophilization, the fluorescent DNA-binding dye of the invention, the DNA-binding dye liquid contains: 1000 µl DNA-binding dye mixed with 25 µl HCl and mixed with 0.1 g cryoprotectant.

### Cryoprotectants

A cryoprotectant in the present context is a substance used to protect biological tissue from freezing damage.

The cryoprotectant according to the present invention, provides bulk to the fluorescent DNA-binding dye freezing formulation especially when the concentration of the product to be frozen dry is very low.

The cryoprotectant according to the present invention, provides adjustments of pH changes during freezing.

The cryoprotectant according to the present invention, protects the product during the freeze-drying against the freezing and drying stresses.

The cryoprotectant according to the present invention, yields an isotonic solution and control the osmotic pressure.

The cryoprotectant according to the present invention, increases the collapse temperature of the product to get higher drying temperatures.

Examples of cryoprotectants are listed in the substance column below

| Type | Function | Substance |
|---|---|---|
| Bulking agents | Provide bulk to the formation | Hydroxyethyl starch, trehalose, mannitol, lactose and glycine |
| Buffer | Adjust pH changes | Phosphate, tris HCL, citrate and histidine |
| Stabilizers | Protect against stress | Sucrose, lactose, glucose, trehalose, glycerol, mannitol, sorbitol, glycine, alanine, lysine, polyethylene glycol, dextran and PVP |
| Tonicity adjusters | Yield an isotonic solution | Mannitol, sucrose, glycine, glycerol and sodium chloride |
| Collapse temperature modifiers | Increase collapse temp | Dextran, hydropropyl-beta-cyclodextrin, PEG, poly(vinyl pyrrolidone) |

In one or more exemplary embodiments, the cryoprotectant is selected from the group consisting of Hydroxyethyl starch, trehalose, mannitol, lactose, glycine, phosphate, tris HCL, citrate, histidine, sucrose, glucose, glycerol, sorbitol, alanine, lysine, polyethylene glycol, dextran, PVP, glycerol, sodium chloride, Dextran, hydropropyl-beta-cyclodextrin, PEG, and poly(vinyl pyrrolidone).

In one or more exemplary embodiments, the cryoprotectant is selected from the group consisting of trehalose, mannitol or mixtures thereof.

### Saccharides

A cryoprotectant according to the invention can be a saccharide. Saccharides limit intracellular dehydration during freezing and protect against denaturation. Moreover, some saccharides stabilize bilayers and preserve membrane function and structure.

In the present context, a cryoprotective saccharide may be selected from the group consisting of fructose, galactose, glucose, rhamnose, xylose, lactose, maltose, sucrose, trehalose, melezitose, raffinose, sorbitol and mannitol or mixtures thereof.

In one or more exemplary embodiments, the cryoprotectant is mannitol.

### Non-saccharide

A cryoprotectant according to the invention, can also be a non-saccharide like for example PEGs (ethylene glycol, PEG 200, PEG 2 000, PEG 20 000), or peptides such as alanine and lysine, or protein fractions generated by enzymatic hydrolysis of for example gelatin by papain or mixtures thereof.

The inventors examined in Example 7 the effects of various cryoprotectant to ascertain which would be the best cryoprotectant for the compressed compositions of the invention, and the results demonstrates that:
Tehalose (range of 10-50 %) - not best formed a paste-like, stretchy and glassy product
Dextran (range of 1.2-25 %) - very hard and insoluble mass product
Trehalose + sucrose (range of 10-25 %+3-10 %) - stretchy and glassy product
Mannitol + sucrose (range of 10 %+3 %) - powdery product is highly soluble
Mannitol (range of 10-18.18 %) - good product, powdery product is highly soluble.

In one or more exemplary embodiments, the cryoprotectants are added in the range of 5-18.18% w/v.

In one or more exemplary embodiments, the cryoprotectants are added in the range of 10-18.18% w/v

In one or more exemplary embodiments, the cryoprotectant is mannitol.

In one or more exemplary embodiments, the cryoprotectant is a combination of mannitol and sucrose.

### Process of manufacture compressed compositions

Further, in one or more exemplary embodiments the invention relates to a method for preparing a compressed composition for use in electrophoretic analysis comprising:
a) providing a blend having a uniform particle size of a polymer matrix particle(s), an electrophoretic conductive medium particle(s), and a lyophilized fluorescent DNA-binding dye, and
b) compressing the blend obtained in step a).

In one or more exemplary embodiments, the method for preparing a compressed composition for use in electrophoretic analysis comprising:
a) providing a blend of a blend of polymer matrix particle(s) and electrophoretic conductive medium particle(s) and a lyophilized fluorescent DNA-binding dye mixture comprising HCL, agarose and mannitol,
b) grinding the blend to a uniform size of 20 µm to 300 µm, and
c) direct compression of the uniform blend obtained in step b) with a maximum force of 15 kg.

Optionally, the lyophilized fluorescent DNA-binding dye mixture comprising HCL, agarose and mannitol can be added after the grinding.

Compressed compositions made by the above method are the most widely used solid dosage form, so they must satisfy a number of physical requirements in terms of:
hardness - easy to pack tablet - yet still the product dissolve fast disintegration ability - no
friability - friction - no
uniformity - no

Compressed compositions made by the above method are receptive to mechanical stress made by a crush tests, i.e. mechanical resistances.

### Humidity during compression

If the humidity of the blend is too low, then the composition is like powder and thus too dusty. Thus, the blend is hard to compress, because the powder accumulates on the punches neck (tooling) and, therefore, aggravates compression and may interrupt tablet weight because the stance (lower tooling) movement is limited.

If the humidity is too high, then it violates the product and its preservation. But not all high humidity makes conductive media and agarose sticky and it stays at punches (upper and lower tooling) at the time of pressing and makes it impossible to operate.
In one or more exemplary embodiments, the relative humidity during compression is between 15-60% humidity
In one or more exemplary embodiments, the relative humidity during compression is between 30-45% humidity
In one or more exemplary embodiments, the blend is left in the room 30 min prior to compression.

### Advantages of direct compression

As described above, the presently preferred compressed composition for use in electrophoretic analysis according to the invention, is prepared by direct compression. The prime advantage of direct compression over wet granulation is economical, since the direct compression requires fewer unit operations. This means less equipment, lower power consumption, less space, less time and less labor leading to reduced production costs of tablets.

Direct compression is more suitable for moisture and heat sensitive APIs, since it eliminates wetting and drying steps and increases the stability of active ingredients by reducing detrimental effects. Changes in dissolution profiles are less likely to occur in tablets made by direct compression on storage than in those made from granulations. This is extremely important because the official compendium now requires dissolution specifications in most solid dosage forms.

Disintegration or dissolution is the rate limiting step in absorption in the case of tablets of poorly soluble API prepared by wet granulation. The tablets prepared by direct compression disintegrate into API particles instead of granules that directly come into contact with dissolution fluid and exhibit comparatively faster dissolution.

The high compaction pressure involved in the production of tablets by slugging or roller compaction, can be avoided by adopting direct compression. The chances of wear and tear of punches and dies are less.

Materials are "in process" for a shorter period of time, resulting in less chance for contamination or cross contamination, making it easier to meet the requirements of current good manufacturing practices. Due to fewer unit operations, the validation and documentation requirements are reduced. Due to the absence of water in granulation, the risk of microbial growth is minimal in tablets prepared by direct compression.

### Photosensitivity

Fluorescent DNA-binding dyes are typically photosensitive, and thus must be stored in non-permeable packaging. However, in the compressed composition, the polymer matrix particles and the electrophoretic conductive medium particles cover the lyophillized fluorescent DNA-binding dye and thus the need for such a packaging is less relevant. The tablet covers the dye molecules from direct light. Consequently, these types of compressed compositions are more stable over time. The compressed compositions can be stored at room temperature.

In one or more exemplary embodiments, the compressed compositions according to the invention can be long term storage without losing quality of the ability of making a high-quality gel electrophoresis. In one or more exemplary embodiments, the compressed compositions according to the invention can be stored more than 8 months without loss of quality.

### Packaging

The compressed compositions according to the invention, is packing in a blister pack with aluminium on top to prevent loss of humidity.

Blister pack is a term for several types of pre-formed plastic packaging used for small consumer goods, foods, and for pharmaceuticals.

The primary component of a blister pack is a cavity or pocket made from a formable web, usually a thermoformed plastic. This usually has a backing of paperboard or a lidding seal of aluminium foil or plastic. A blister that folds onto itself is often called a clamshell.

Blister packs are useful for protecting products against external factors, such as humidity and contamination for extended periods of time.

### Multicomponent tablet

It is an object of the present invention to provide a method of saving work and time in a manner different from the prior art for performing a high-quality and efficient DNA/RNA analysis and preparing a mixture of electrophoresis agarose gel used in DNA/RNA analysis.

The object of the invention is achieved by a multicomponent compressed powder mixture prepared according to the present method, said multicomponent compressed powder mixture comprising all the components necessary for the synthesis of agarose gel for DNA/RNA analysis, and said multicomponent compressed powder mixture dissolves rapidly in agarose gel formation, is of good quality, the dye of which is highly bind to the DNA and has a low background, and to form an agarose gel dissolved in water. The tablet prepared by this method ensures the formation of a fast and high-quality agarose gel. Since the tablet, according to the present method, contains all the components necessary for DNA/RNA analysis, there is no need for separate components to be considered, making it possible to make agarose gel much easier and eliminating the potential weighing errors. Unlike the solutions known to date, in which defects can also occur in the calculation of the required amount of agarose gel components, the corresponding calculation errors are eliminated, knowing the tablet weight of the present method and, depending on how much liquid is needed, to produce the desired amount and concentration of the agarose gel.

The dye used in the tablet prepared according to this method allows DNA to be visualized with high efficiency and low background activity. DNA-binding dye is photosensitive, and according to well-known solutions, the binding dye of the DNA used must be packaged in non-permeable light in the package. In this method, when the binding dye of the DNA is compressed together with other components to the tablet, the need for such a packaging is less because the other components of the tablet covers the dye molecules from direct light. Consequently, these types of tablets are also very stable over time and, and due to the properties of the components they can also be stored at room temperature.

Freeze-dry DNA dye is further used to produce a tablet for preparing an agarose gel, according to the method, and has not previously been used as an agarose gel in a multicomponent tablet formulation.

Unlike the solutions known so far, the multicomponent tablet used to prepare the agarose gel for the preparation of a tablet was not used as the additive substances (excipients) and can affect the movement of DNA or RNA in agarose gel and therefore the direct compression methodology is used to obtain the tablet using this method. This methodology requires good binding properties of the substances.

Buffers used to produce agarose gel consist of 3 different substances, all of which are flakes of different sizes and weights. This in turn reduces their uniform mixing and tablet moulding capabilities, which in turn reduces the quality. Therefore, it is necessary to perform a uniformization of the size of the various components of the buffer, which improves the binding of the substance to the tablet and the quality of the agarose gel produced from the tablet. Therefore, the size of the agarose component in the tablet formulation according to the present invention, should be similar to that of the buffer composition, since it is otherwise difficult to obtain a homogeneous composition and tablet weight. A non-uniform size of the components in the tablet affects the efficacy of tablet formation. Since very small powder particles do not form a tablet without additives, or the tablet properties do not meet the requirements for the application, for example they may be insoluble.

Unlike the solutions so far known, powdered TBE and TAE buffers, in combination with agarose powder and DNA-binding dye, have according to the invention been used in a multicomponent tablets prepared to form an agarose gel tablet according to the method of the present invention in order to ensure the quality and efficacy of the present invention.

The present invention includes a method of preparing agents for DNA/RNA analysis, multicomponent tabletting, and agarose gel used in the DNA/RNA analysis from a tablet in an easy and cost-effective manner.

The method of the present invention comprises the steps of:
Preparation of the substances to be used (DNA-binding dye);
Physical modification of the existing substances to be used for tablet pouring (TBE and TAE powder buffers);
Weighing of used substances;
Mixing of substances used;
Multi-component tablet press.

Unlike the solutions known to date, the granulation, spray-drying or crystallization of dye substances is not used in the method according to the present invention, but it involves dry-freezing of the dye substances.

The multicomponent tablet for agarose gel of the present invention comprises a fluorescent lyophilized DNA-binding dye, an agarose, an agarose mixed with a DNA binding protein, a cryoprotective agent, and an acrylic buffer (in alternative embodiments of the invention, for example, TBE or TAE).

A method according to the present invention for preparing a multicomponent tablet to form an agarose gel in a DNA/RNA assay comprises the preparation of a DNA-binding dye in powder form; buffering and DNA-binding dye; weighing and mixing the components, and compressing the tablet.

The first step in the preparation is to make DNA-binding dye in powder form.

In gel electrophoresis, a DNA binding dye is a fluorescent substance (dye) that binds to DNA while DNA moves along a gel, and the DNA becomes visible in certain light (e.g., UV, Blue light) for visualization, a variety of light sources can be used.

In the binding of the DNA, cryoprotectants (e.g. Trehalose, Sucrose, Glucose, Mannitol, etc.) are added in the range of 5-10% w / v. The resulting solution was frozen -80 ° C and dry-frozen at 0.07-0.0011 mbar for 17 hours. The resulting solid was mixed with agarose powder (0.8 % of the final volume) and grinded for 2 minutes.

Phase two - Physical modification of existing substances to be suitable for tabletting (TBE and TAE powder buffers).

The TBE or TAE buffer was finely grinded until the granule size ranged from 20-200µm.

Phase three - Weighing of used substances.

Powder DNA binding dye, buffer and agarose are weighed accurately. According to the ratios described below (Example 1, Example 2).

Phase four - Mixing of substances to be used.

Each precision weighed agent is admixed individually in accordance with the ratio described below (Example 1, Example 2) and blended into a homogeneously mixed mixture (the time is 30 minutes per 1 kg of a mixture of substances).

Phase five - Tablet compression step.

Using direct compression, i.e. the substances described in Examples 1 and 2, there are no substances used to compress the tablet and improve the flow of the substance. Pre-granulation of substances has also not been used.

In order to more clearly express the invention, the following examples of the tablet manufacturing method, wherein the concentration ranges are shown, are listed below.

### General

It should be understood that any feature and/or aspect discussed above in connections with the compressed compounds according to the invention apply by analogy to the methods described herein.

The terms compressed compounds and tablet, are used interchangeably.

The following figures and examples are provided below to illustrate the present invention. They are intended to be illustrative and are not to be construed as limiting in any way.

### BRIEF DESCRIPTION OF THE FIGURES

*Figure 1*
   Shows the easy way of how to make 1% agarose gel for gel electrophoresis from compressed composition (tablet) with an electrophoretic conductive medium TBE or TAE. To make 1 % agarose gel, simply take an empty bottle or flask, pour 50 ml of room temperature water into the bottle, take one compressed composition out of the blister pack and place it in the bottle and let the compressed composition soak for 1-3 min prior heating. Pour the gel into the gel casting tray.
*Figure 2*
   Compressed composition in uniform size particles and different particle sizes. As seen on the lower tablet, which is the uniform sized tablet of the invention, it is a complete tablet without cracks and missing parts, thus having the right size/weight. In contrast to the upper tablet, which shows sign of breaking, thus it no longer has the right size/weight.
*Figure 3*
   Trehalose was tested as a cryoprotectant in different concentration range (5-50 % w/v). Regardless of the trehalose concentration, trehalose and fluorescent DNA binding dye always formed a paste-like, stretchy and glassy product during the lyophilization process. This kind of lyophilized fluorescent DNA binding dye was impossible to mill and mix evenly in a compressed composition. Thus, trehalose was considered less suitable as a cryoprotectant for optimal compressed compositions.
*Figure 4*
   Dextran was tested as a cryoprotectant in different concentration range (1,2-25 % w/v). Regardless of the dextran concentration, dextran and fluorescent DNA binding dye always formed a very hard and insoluble mass product during the lyophilization process, suggesting that the combination of dextran and used fluorescent DNA binding dye is not chemically suitable for the lyophilization or the chosen physical conditions (pressure, temperature) caused the structural collapse of the mixture. That kind of lyophilized fluorescent DNA binding dye is impossible to mill and mix evenly in the compressed composition mixture. Thus, dextran was considered less suitable as a cryoprotectant for optimal compressed compositions.
*Figure 5*
   Mannitol was tested as a cryoprotectant in at10 % w/v in a small amount of fluorescent DNA binding dye (200 µl). Mannitol and fluorescent DNA binding dye formed a powdery, easy to mill and well dissolving product during the lyophilization process, thus in the chosen physical conditions (pressure, temperature) water was removed from the fluorescent DNA binding dye without destructing its structure. This combination of lyophilized fluorescent DNA binding dye and mannitol resulted in products that was easy to mill and mix evenly in the blend and prepare suitable compressed compositions.
*Figure 6*
   Mannitol was tested as a cryoprotectant in at10 % w/v in a larger amount of fluorescent DNA binding dye (5000 µl). Mannitol and fluorescent DNA binding dye still formed a powdery, easy to mill and well dissolving product during the lyophilization process. thus in the chosen physical conditions (pressure, temperature) water was removed from the fluorescent DNA binding dye without destructing its structure. This combination of lyophilized fluorescent DNA binding dye and mannitol resulted in products that was easy to mill and mix evenly in the blend and prepare suitable compressed compositions.
*Figure 7*
   Show the results of a photosensitivity test. Tablets were stored at room temperature for 8 months protected from light. Tablet is used for making agarose gel at the parameters 1 % agarose gel and running parameters: 150 V, 45 min and in 1x TBE buffer. The image was taken using a The Blue Light Transilluminator. The gel was loaded with DNA ladder - 1kb Solis DNA ladder - from left to right 2, 4, 6 or 8 µl respectfully. The quality of the gel and the image is high and the migrated DNA bands are strong and sharp, and the background intensity low - despite the long-term storage of the tablet for 8 months.
*Figure 8*
   Show the results of a photosensitivity test. Tablets were stored at room temperature for 8 months without protection from light (placed in direct sunlight). Tablet is used for making agarose gel at the parameters 1 % agarose gel and running parameters: 150 V, 45 min and in 1x TBE buffer. The image was taken using a The Blue Light Transilluminator. The gel was loaded with DNA ladder - 1kb Solis DNA ladder - from left to right 2, 4, 6 or 8 µl respectfully. The quality of the gel and the image is high and the migrated DNA bands are strong and sharp, and the background intensity low - despite the long-term storage of the tablet for 8 months without without protection from sunlight. This shows that the compressed composition in itself provides protection for the photosensitive lyophilized fluorescent DNA binding dye.
*Figure 9*

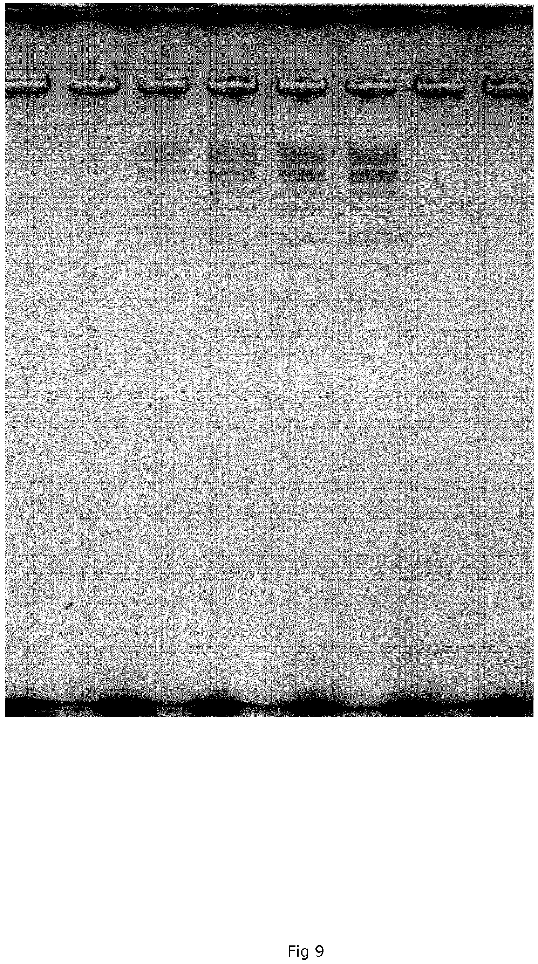

   Figures 9-11 show the effect if the lyophilized fluorescent DNA-binding dye is not milled/grinded, where figure 9 is a positive control or well-stained gel with milled lyophilized fluorescent DNA-binding dye.
   The images 9-11 was made using tablets according the invention with or without milling/grinding and the following parameters were used for making the agarose gel: 1 % agarose gel and running parameters: 150 V, 45 min and in 1x TBE buffer. The images was taken using a The Blue Light Transilluminator. The gels were loaded with DNA ladder - 1kb Solis DNA ladder - from left to right 2, 4, 6 or 8 µl respectfully.
*Figure 10*

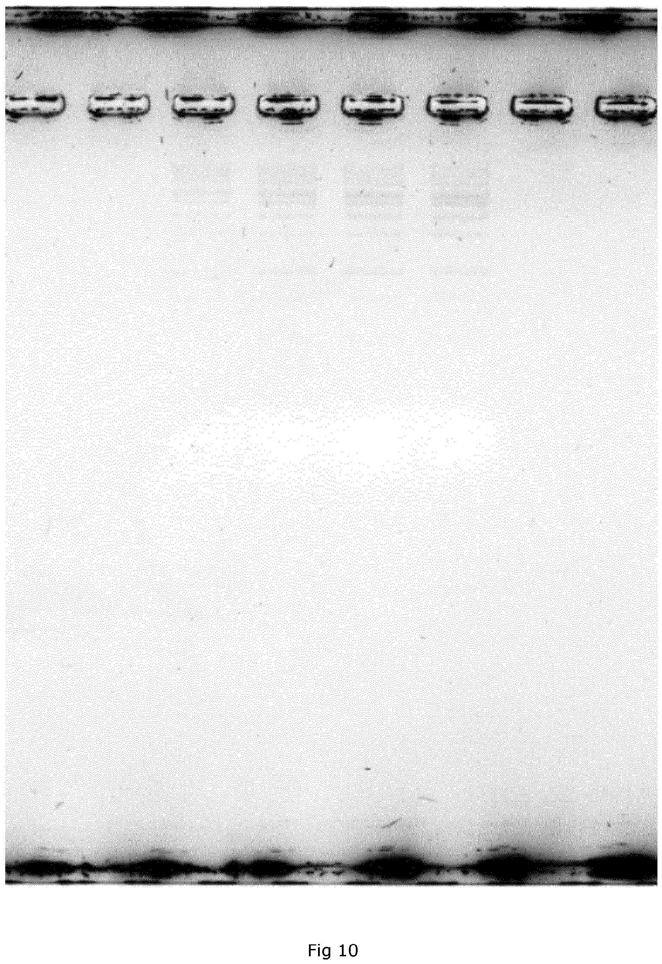

   Figure 10 is an under stained negative gel with lyophilized fluorescent DNA-binding dye without a milling or a grinding process.
*Figure 11*

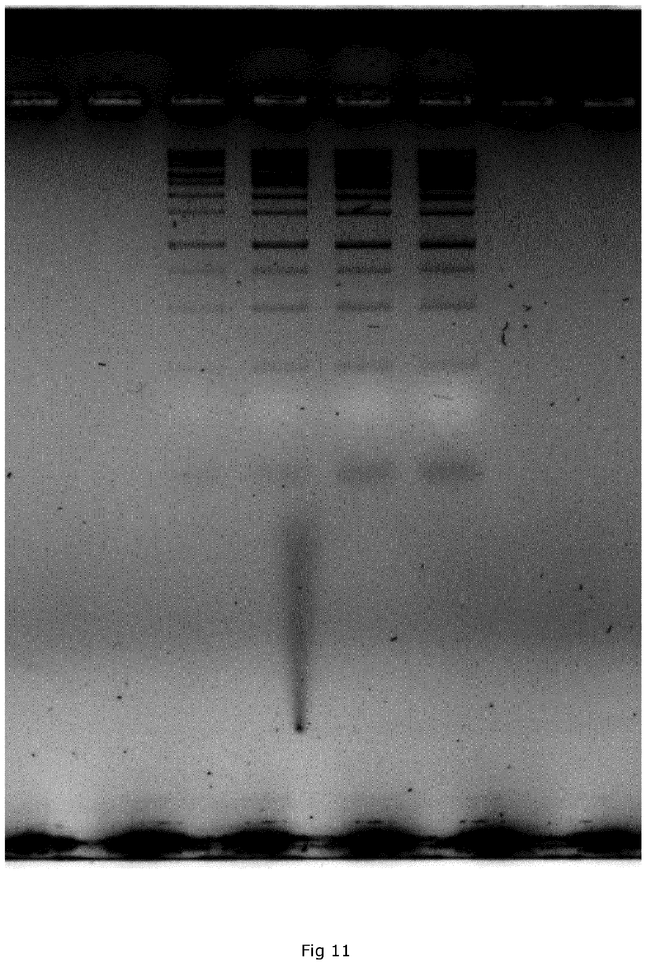

   Figure 11 is an over stained negative gel with lyophilized fluorescent DNA-binding dye without a milling or grinding process.

### EXAMPLES

### Example 1 - TBE buffer components

| Used substance | Mass of the substance expressed as% |
|---|---|
| Powder DNA binding dye | 0.004 % |
| Agarose I (mixed with DNA-binding agent) | 0.8 % |
| Grinded TBE buffer | 50 % |
| Agarose II | 49.2 % |

### Description of the exercise:

1) In the binding of the DNA, cryopreservation agents were added in the range of 5-10 % w/v. The resulting solution was frozen -80 ° C and dry-frozen at 0.07-0.0011 mbar for 17 hours.
2) The resulting solid was mixed with Agarose I powder (0.8 % of final volume) and peeled off for 2 min until the granule size ranged from 20-200 µm.
3) The components of the TBE buffer were triturated for 3 seconds until the granule size ranged from 20-200 µm.
4) Powder DNA binding binder 5 and agarose II mixture (0.8 %) were mixed with the rest of the agarose (49.2 %) and stirred for 5 min using the appropriate stirrer. The finely divided TBE powder was then added to the mixture of remaining substances and stirred for an additional 30 minutes (counting 30 min for 1 kg of mixed components) using a suitable stirrer (e.g., using a roller mixer)
5) Pressing the tablet

### Tablet characteristics:

| | |
|---|---|
| Size | 10x20x6 mm |
| Shape | Oval, without folding |
| Weight | 1g |
| Strength (force used by the press) | 15 kg |
| Solubility | Less than 3 min |

### Example 2 - TAE buffer components

| Used substance | Mass of the substance expressed as% |
|---|---|
| Powder DNA binding dye | 0.004 % |
| Agarose I (mixed with DNA-binding agent) | 0.8 % |
| Grinded TBA buffer | 35 % |
| Agarose II | 64.2 % |

### Description of the exercise:

1) In the binding of the DNA dye, cryopreservation agents were added in the range of 5-10 % w/v. The resulting solution was frozen -80 ° C and subsequently dry-frozen at 0.07-0.0011 mbar for 17 hours.
2) The resulting solid was mixed with Agarose I powder (0.8 % of final volume) and grinded for 2 min until the granule size ranged from 20-200 µm.
3) The components of the TAE buffer were grinded for 3 seconds until the pellet size ranged from 20-200 µm.
4) Powder DNA-binding dye and Agarose II mixture (0.8 %) were mixed with the rest of the agarose (64.2 %) and stirred for 5 min using the appropriate stirrer. The peeled TAE powder was then added to a mixture of the remaining substances and stirred for an additional 30 minutes (counting 30 min per 1 kg of mixed components) using a suitable mixer (e.g., using a roller mixer).
5) Pressing the tablet
6) Tablet characteristics:

| | |
|---|---|
| Size | 10x20x6 mm |
| Shape | Oval, without folding |
| Weight | 1g |
| Strength (force used by the press) | 15 kg |
| Solubility | Less than 3 min |

According to this method, the electrophoresis agarose gel is formed from the tablet prepared according to the present method as follows: In the gel preparation vessel, the required number of tablets and the corresponding amount of warm water used to make the gel are added. The tablet(s) is/are dissolved in 3 minutes. Visually inspect the whole powder of the tablet to dissolve in the water before heating the gel. An example of making a gel for DNA/RNA analysis is described in the table.

| Gel % | 1 tablet | 2 tablets |
|---|---|---|
| 1 % | 50 ml room temp water | 100 ml room temp water |
| 1.5 % | 33 ml room temp water | 67 ml room temp water |

The solution is heated until it has become transparent and all visible particles are dissolved. Then cool the gel solution to 60°C before pouring it on the base. The gel is then run in the appropriate buffer with the desired parameters.

### Example 3 - Atlas ClearSight Tablet use with TBE

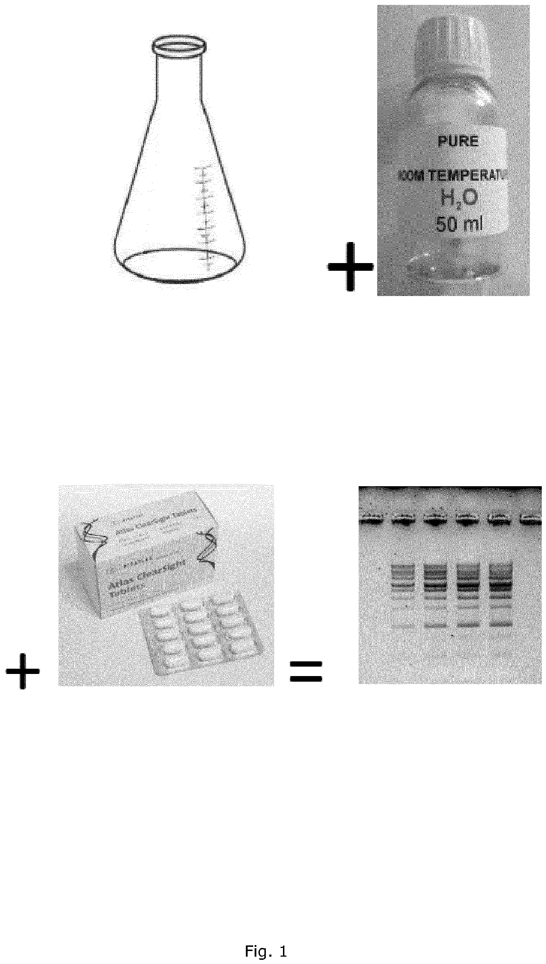

To make 1 % agarose gel for gel electrophoresis, take one compressed composition (tablet) with electrophoretic conductive medium TBE out of the blister pack. Place it in the bottle or flask and add 50 ml of room temperature water. Soak the compressed composition for 1-3 minutes. Heat solution until all particles are dissolved. Pour the gel out into the gel casting tray, see Figure 1.

As only water needs to be added to the compressed composition (tablet) to make the agarose gel for electrophoresis, it makes it much faster and easier to make the agarose gel compared to preparing agarose gel out of separate components, which need to be weighed.

### Example 4 - Atlas ClearSight Tablet use with TAE

To make 1.3 % agarose gel for gel electrophoresis, take one compressed composition (tablet) with electrophoretic conductive medium TAE out of the blister pack. Place it in the bottle or flask and add 50 ml of room temperature water. Soak the compressed composition for 1-3 minutes. Heat solution until all particles are dissolved. Pour the gel out into the gel casting tray, see Figure 1.

As only water needs to be added to the compressed composition (tablet) to make the agarose gel for electrophoresis, it makes it much faster and easier to make the agarose gel compared to preparing agarose gel out of separate components, which need to be weighed.

### Example 5 - Atlas ClearSight Agarose tablets without electrophoretic conductive medium

To make 1 % agarose gel for gel electrophoresis take one compressed composition (tablet) without electrophoretic conductive medium out of the blister pack, place it in the bottle or flask and add 25 ml of electrophoretic conductive medium solution. Soak the compressed composition for 1-3 minutes. Heat solution until all particles are dissolved. Pour the gel out into the gel casting tray.

### Example 6 - Uniform ingredients in the blend

Two types of tablet where produced by direct compression with the following blends:
- 40 to 75 wt% agarose powder;
- 25 to 60 wt% DNA electrophoretic conductive medium;
- less than 0.05 wt% lyophilized fluorescent DNA-binding dye
with or with-out grinding the blends.

The compressed composition comprising the grinded blend is intact, and its shape is preserved. In the subsequent use, the buffer properties are maintained since all the components are in the right proportions. Also, the tablet weight is maintained, since all the components are in the right proportions.

The compressed composition comprising the blend, which was not grinded shows signs of cracks, and its shape is not preserved. In the subsequent use, the buffer properties were not maintained, since all the components were not in the right proportions.

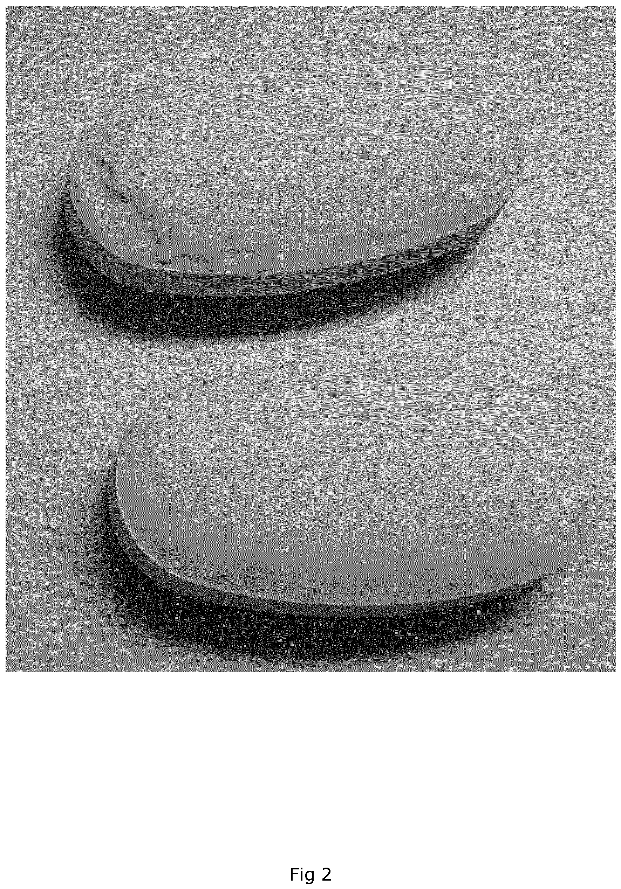

Therefore, the size of the polymer matrix particle component in the compressed composition according to the invention should be similar or uniform to that of the electrophoretic conductive medium particle, since it is otherwise difficult to obtain a homogeneous composition. A non-uniform size of the components in the compressed composition affects the efficacy of tablet formation. For example, if the blend is not non-uniform during the compression, the form of compressed composition (tablet) can be broken or it may not be formed to the correct shape at all, see Figure 2.

### Example 7 - Cryoprotectants

Tablets produced by direct compression with the following blends:
- 40 to 75 wt% agarose powder;
- 25 to 60 wt% DNA electrophoretic conductive medium;
- less than 0.05 wt% lyophilized fluorescent DNA-binding dye
with different cryoprotectants.

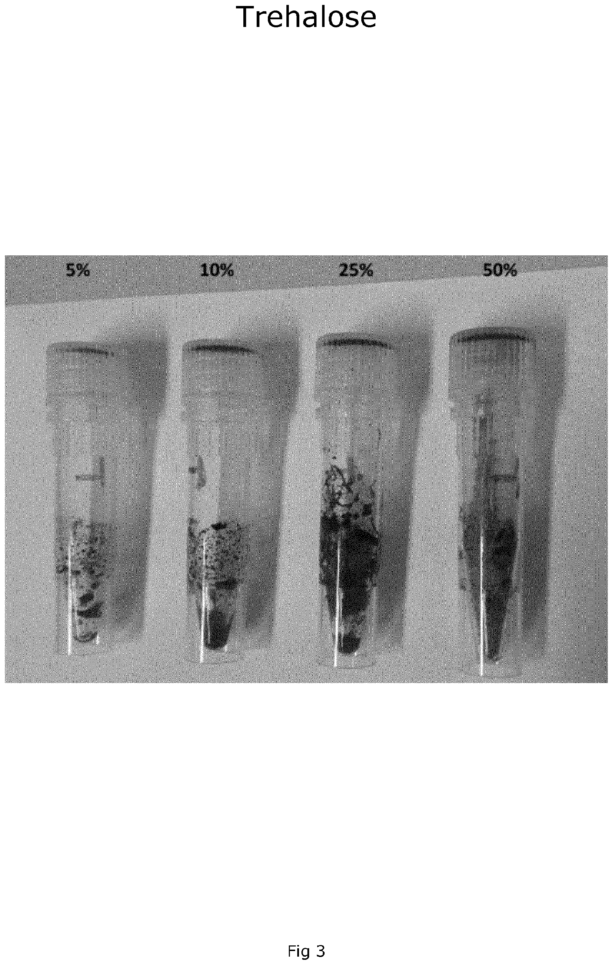

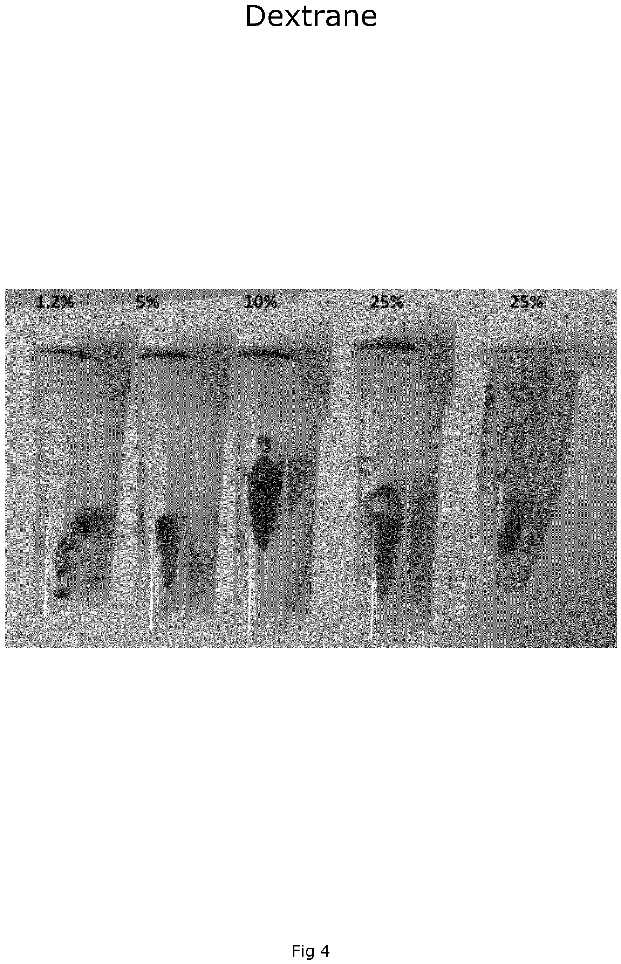

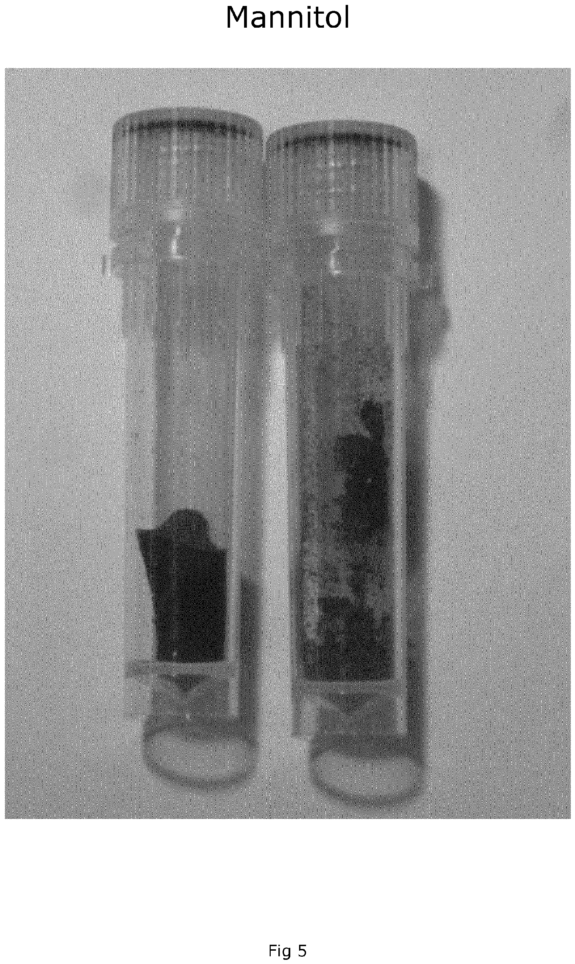

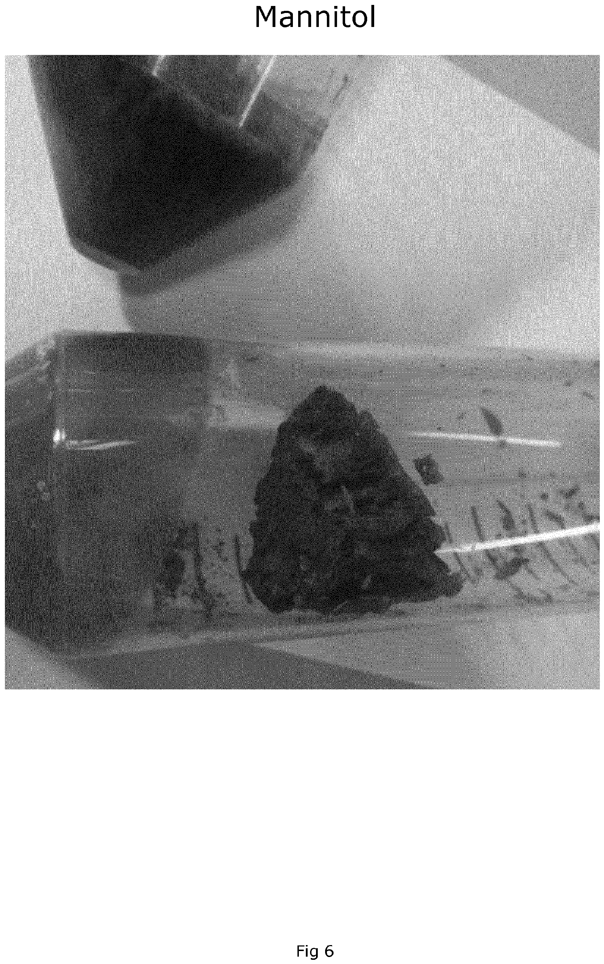

Here is the list and the results of those cryoprotectants which were tested:
A) Glycerol does not work in any concentration range because it is impossible to lyophilize due to its oily-like chemical structure,
B) Trehalose (tested in the range of 5-50 % w/v). Despite the trehalose concentration, trehalose and fluorescent DNA binding dye always formed a paste-like, stretchy and glassy product during the lyophilization process. This means that either the combination of trehalose and used fluorescent DNA binding dye is not chemically suitable for the lyophilization or the chosen physical conditions (pressure, temperature) caused the structural collapse of the mixture. Such a lyophilized fluorescent DNA binding dye is impossible to mill and mix evenly in the compressed composition mixture (See for example Figure 3).
C) Dextran (tested in the range of 1.2- 25 % w/v). Despite the dextran concentration, dextran and fluorescent DNA binding dye always formed a very hard and insoluble mass product during the lyophilization process and thus were impossible to mill and mix evenly in the compressed composition mixture.
   (See for example Figure 4),
D) Trehalose + sucrose (tested in the range of 10-25 % w/v +3-10 % w/v) does not work, stretchy and glassy product (data not shown), and
E) Mannitol (tested in the range of 10-18.18 % w/v). Mannitol and fluorescent DNA binding dye formed a powdery, easy to mill and well dissolving product during the lyophilization process despite of the amount of the fluorescent DNA binding dye used in lyophilization process. Thus, this tested cryoprotectant in range of 10-18.18 % w/v was considered extremely suitable (See Figures 5 and 6).

### Example 8 - Efficiency gain in lab time

It is easier and faster to make the agarose gel for electrophoresis if only water needs to be added to the compressed composition (tablet) compared to preparing agarose gel out of separate components, which need to be weighed.

| Activities | The compressed composition (tablet) | With separate components |
|---|---|---|
| Polymer matrix particle component weighting | - | 2 min 35 sec |
| Electrophoretic conductive medium weighting | - | 2 min 25 sec |
| Adding water | 30 sec | 30 sec |
| Dissolving time | 1 min | - |
| Melting time | 3 min | 3 min |
| Time to cool | No need | 2 min |
| Adding fluorescent DNA binding dye | - | 1 min |
| Total | 4 min 30 sec | 11 min 30 sec |

### Example 9 - Dust wipe

Two gels were prepared, one by use of a compressed composition in 4 min and 30 sec, as shown in Example 8, and a second gel by use of separate components in 11 min and 30 sec. After the preparation, the work desk was wiped with a dust wipe and inspected for visual dust. The wiping was made in one direction only, not back and forth.

The work desk used for both preparations was cleaned prior to the test with a High Efficiency Particulate Air (HEPA) vacuum cleaner.

The work table used for preparing an agarose gel from with a compressed composition (tablet), did not show any dust in the visual inspection.

The work table used for preparing an agarose gel from separate components, showed significant dust in the visual inspection (data not shown).

### Example 10 - Precision of the compressed compositions

For making gel out of a powdery polymer matrix particle component and electrophoretic conductive medium, two separate weighs must be performed. This leads to different weighing errors. At first there are 2 scale errors taking place due to the 2-step weighing (usually the scale error is 0.01 % per weighing). And then some part of the weighed material (usually 0.5-1 %) remain(s) in the weighing container, causing an additional error to occur.

Thus 2 scale errors may take place. A human error coming from wrong weighing or rounding the numbers is approximately 2 %. The additional error coming from preparing the electrophoretic conductive media solution (error from beaker/measuring glass, human error, rounding error) is around 1-4 %.

Adding fluorescent DNA-binding dye to the electrophoretic conductive media solution causes another error, of which the pippet error is 0.001 % and pipetting error may be up to 5%.
2 weighing errors may take place:
Human error- wrong weighting or rounding error (2 %)
For making electrophoretic conductive media- fluid error (error from beaker/measuring glass,
human error, rounding error) 1-4 %.
Adding fluorescent DNA binding dye- Pipette error (0.001%) and pipetting error (up to 5%).

### Example 11 - Easy to use protocol TBE

Use the bottle or flask that is at least 3 times of the volume of the solution being prepared. For tablet dissolving use water which is at room temperature, do not use hot water. Add an appropriate number of agarose tablets in the water and do not add any buffer. See the table below to achieve needed gel percentage.

| Gel % | 1 tablet | 2 tablets |
|---|---|---|
| 1% | 50 ml water | 100 ml water |
| 1.5% | 33 ml water | 67 ml water |

Soak the tablet in the water for 3 minutes (or until it is dissolved) before heating.

Heat the solution until it is clear and visually all particles are dissolved.

Cool the solution to approximately 60° C before pouring it out.

Run the gel in the TBE running buffer.

### Example 12 - Easy to use protocol TAE

Use the bottle or flask that is at least 3 times of the volume of the solution being prepared. For tablet dissolving use water which is at room temperature, DO NOT use hot water.

Add an appropriate number of tablets in the water and do NOT add any buffer. See the table below to achieve the needed gel percentage.

| Gel % | 1 tablet | 2 tablets |
|---|---|---|
| 1% | 65 ml water | 130 ml water |
| 1.5% | 43 ml water | 86 ml water |

Soak the tablet in the water for 3 minutes (or until it is dissolved) before heating.

Heat the solution until it is clear and visually all the particles are dissolved.

Cool the solution to approximately 60° C before pouring it out.

Run the gel in the TAE running buffer.

### Example 13 - Photosensitivity

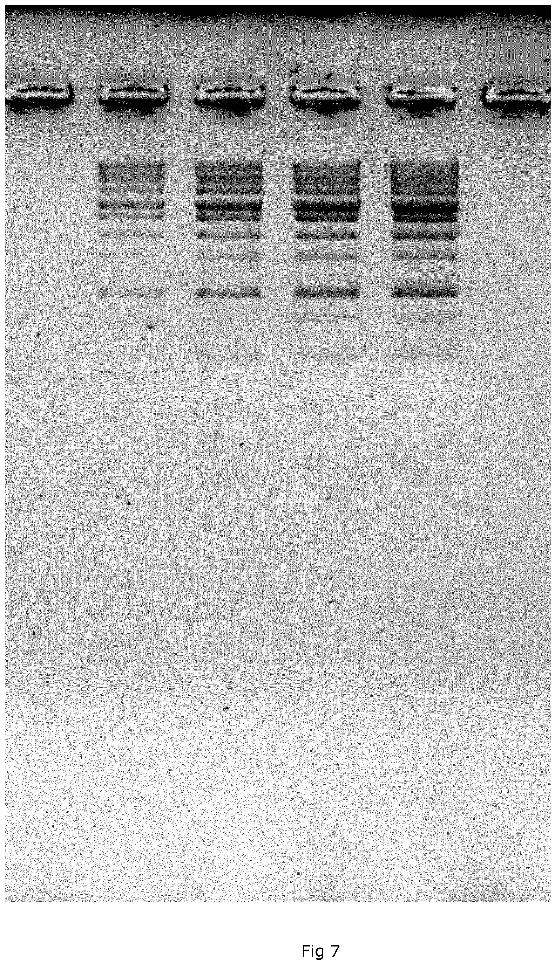

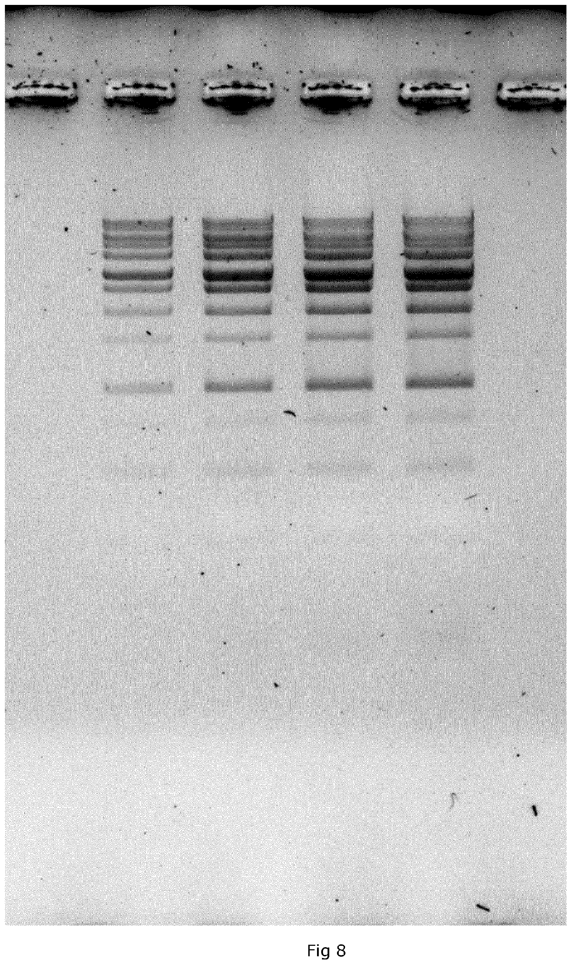

Fluorescent DNA-binding dye is typically photosensitive, and thus must be stored in non-permeable packaging. However, the in the compressed composition, the polymer matrix particles and the electrophoretic conductive medium particles cover the lyophillized fluorescent DNA-binding dye and thus the need for such a packaging is less relevant. The tablet covers the dye molecules from direct light. Consequently, these types of compressed compositions are more stable over time. The compressed compositions can be stored at room temperature (Figure 7 and 8).

### Example 14- Lyophillized fluorescent DNA-binding dye milling/grinding

For pre-compressing the compressed composition, one important step is milling the lyophillized fluorescent DNA-binding dye. Otherwise the lyophillized fluorescent DNA-binding dye will not be evenly distributed in that compressed composition, which results in an uneven quality of agarose gel electrophoreses (some agarose gels are over stained, and some are under stained due to a non-optimal concentration of fluorescent DNA-binding dye) (see Figures 9, 10 and 11).

## Claims

1. A compressed composition for use in electrophoretic analysis comprising a blend of polymer matrix particles comprising agarose and a buffer made from electrophoretic conductive medium particles, wherein the blend has a uniform particle size, a lyophilized fluorescent DNA-binding dye and a cryoprotectant.

2. A compressed composition according to claim 1, wherein the blend has a uniform particle size between 20-300 µm.

3. A compressed composition according to any of the preceding claims, wherein the blend comprises between:
- 40 to 75 wt % matrix particles comprising agarose;
- 25 to 60 wt % DNA electrophoretic conductive medium particles;
- less than 0.01 wt % lyophilized fluorescent DNA-binding dye.

4. A method for preparing a compressed composition for use in electrophoretic analysis comprising:
a) providing a blend having a uniform particle size of polymer matrix particles comprising agarose, electrophoretic conductive medium particles, a lyophilized fluorescent DNA-binding dye and a cryoprotectant, and
b) compressing the blend obtained in step a).

5. A method for preparing a lyophilized dye for use in a compressed composition according to claims 1-3 comprising:
a) providing a fluorescent DNA-binding dye in liquid format and a cryoprotectant, and
b) lyophilization the fluorescent DNA-binding dye and cryoprotectant by use of low temperature, pressure and sublimation.

## Patentansprüche

1. Komprimierte Zusammensetzung zur Verwendung in der elektrophoretischen Analyse, umfassend eine Mischung aus Polymermatrixpartikeln, die Agarose umfassen, und einem Puffer aus Partikeln eines elektrophoretischen leitfähigen Mediums, wobei die Mischung eine einheitliche Partikelgröße, einen lyophilisierten fluoreszierenden DNA-bindenden Farbstoff und ein Kryoschutzmittel aufweist.

2. Komprimierte Zusammensetzung nach Anspruch 1, wobei die Mischung eine einheitliche Partikelgröße zwischen 20-300 µm aufweist.

3. Komprimierte Zusammensetzung nach einem der vorhergehenden Ansprüche, wobei die Mischung Folgendes umfasst:
- zwischen 40 und 75 Gew.-% Matrixpartikel, die Agarose umfassen;
- zwischen 25 und 60 Gew.-% elektrophoretisch leitfähige DNA-Mediumpartikel;
- weniger als 0,01 Gew.-% lyophilisierten fluoreszierenden DNA-bindenden Farbstoff.

4. Verfahren zum Herstellen einer komprimierten Zusammensetzung zur Verwendung in der elektrophoretischen Analyse, umfassend:
a) Bereitstellen einer Mischung mit einer einheitlichen Partikelgröße aus Polymermatrixpartikeln, die Agarose, Partikel eines elektrophoretisch leitfähigen Mediums, einen lyophilisierten fluoreszierenden DNA-bindenden Farbstoff und ein Kryoschutzmittel umfasst, und
b) Komprimieren der in Schritt a) erhaltenen Mischung.

5. Verfahren zum Herstellen eines lyophilisierten Farbstoffs zur Verwendung in einer komprimierten Zusammensetzung nach Ansprüchen 1-3, umfassend:
a) Bereitstellen eines fluoreszierenden DNA-bindenden Farbstoffs in flüssiger Form und eines Kryoschutzmittels, und
b) Lyophilisierung des fluoreszierenden DNA-bindenden Farbstoffs und des Kryoschutzmittels durch Verwendung von niedriger Temperatur, Druck und Sublimation.

## Revendications

1. Composition comprimée destinée à être utilisée dans l'analyse électrophorétique comprenant un mélange de particules de matrice polymère comprenant de l'agarose et un tampon constitué de particules de milieu conducteur électrophorétique, dans laquelle le mélange a une taille de particules uniforme, un colorant fluorescent lyophilisé se liant à l'ADN et un cryoprotecteur.

2. Composition comprimée selon la revendication 1, dans laquelle le mélange a une taille de particules uniforme comprise entre 20 et 300 µm.

3. Composition comprimée selon l'une quelconque des revendications précédentes, dans laquelle le mélange comprend entre :
- 40 à 75 % en poids de particules de matrice comprenant de l'agarose ;
- 25 à 60 % en poids de particules de milieu conducteur électrophorétique d'ADN ;
- moins de 0,01 % en poids de colorant fluorescent lyophilisé se liant à l'ADN.

4. Procédé de préparation d'une composition comprimée destinée à être utilisée dans l'analyse électrophorétique comprenant :
a) la fourniture d'un mélange ayant une taille de particules uniforme de particules de matrice polymère comprenant de l'agarose, des particules de milieu conducteur électrophorétique, un colorant fluorescent lyophilisé se liant à l'ADN et un cryoprotecteur, et
b) la compression du mélange obtenu à l'étape a).

5. Procédé de préparation d'un colorant lyophilisé destiné à être utilisé dans une composition comprimée selon les revendications 1 à 3 comprenant :
a) la fourniture d'un colorant fluorescent se liant à l'ADN sous forme liquide et d'un cryoprotecteur, et
b) la lyophilisation du colorant fluorescent se liant à l'ADN et du cryoprotecteur en utilisant une basse température, pression et sublimation.
